# EUROPÄISCHE PATENTANMELDUNG

(11) **EP 2 447 397 A1**
(43) Veröffentlichungstag der Anmeldung: **02.05.2012**
(21) Anmeldenummer: 10014110.0
(22) Anmeldetag: 29.10.2010
(51) Int. Cl.: D01D 5/18, D01F 1/10, D01F 6/62, D01F 6/68, D01F 6/82, A61L 15/22, D04H 1/72

(54) **Vliesstoffe aus synthetischen Polymeren sowie Rotationsspinnverfahren zur Herstellung derselben**

(71) Anmelder: Carl Freudenberg KG, 69469 Weinheim (DE)
(72) Erfinder: Reibel, Denis, Dr., 67850 Herrlisheim (FR); Grafahrend, Dirk, 68165 Mannheim (DE)

(57) **Zusammenfassung**

Ein Vliesstoff, umfassend Fasern aus einem Faserrohmaterial, wobei die Fasern zumindest eine biologisch aktive Substanz aufweisen, die zumindest teilweise in und/ oder auf den Fasern angeordnet ist, ist im Hinblick auf die Aufgabe, einen bioabbaubaren Vliesstoff anzugeben, die sich durch eine höhere mechanische Stabilität und durch eine kostengünstigere Herstellung auszeichnet, dadurch gekennzeichnet, dass das Faserrohmaterial als polymeren Bestandteil zumindest ein synthetisches Polymer in unmodifizierter und/ oder modifizierter Form aufweist, wobei das synthetische Polymer biologisch abbaubar und/ oder biokompatibel ist.

## Beschreibung

### Technisches Gebiet

Die Erfindung betrifft einen Vliesstoff gemäß dem Oberbegriff des Patentanspruchs 1.

### Stand der Technik

Vliesstoffe, die mit biologisch aktiven Substanzen ausgerüstet sind, sind bereits aus dem Stand der Technik bekannt. Insbesondere zeigt die WO 2004/002384 A1 einen Vliesstoff, welcher Silber aufweist und als Wundauflage verwendet wird. Als Faserrohmaterialien werden dort Polyurethane oder Polyacrylate vorgeschlagen.

Vliesstoffe werden häufig für medizinische Anwendungen verwendet. Den als Rohmaterial für die Vliesstoffe vorliegenden Vliesen wird durch Wasserstrahlverfestigung, thermische oder chemische Verfestigung eine hinreichende Reißfestigkeit verliehen, damit diese als Verbandsmaterial eingesetzt werden können.

Die mechanischen oder chemischen Verfestigungsverfahren können jedoch die Ausrüstung der Fasern mit biologischen Wirkstoffen, beispielsweise antimikrobiellen oder antibiotischen Wirkstoffen, negativ beeinträchtigen, nämlich die wirksamen Stoffe in ihrer Wirkung hemmen oder sogar teilweise aus den Fasern herauslösen.

Daher sind häufig aufwendige und teure Nachbehandlungsschritte notwendig, um die verfestigten Vliesstoffe in einen gebrauchstauglichen Zustand zu verbringen.

Für die Aufbringung biologisch aktiver Substanzen auf Vliesstoffe werden häufig Verfahren verwendet, bei denen der Vliesstoff nach seiner Herstellung mit den Substanzen behandelt wird. Dies hat den Nachteil, das mehrstufige Verfahren erforderlich sind, bei denen die Herstellung des Vliesstoffes gesondert von der Aufbringung der Substanzen erfolgt.

Zudem wird bei solchen Verfahren regelmäßig nicht eine gleichmäßige, stabile Verteilung der Substanzen in dem Vliesstoff erzielt. Daher müssen oft Ärzte oder medizinisches Personal die Aufbringung der aktiven Substanzen auf die industriell hergestellten Vliesstoffe vornehmen.

Ein weiteres Problem ist, dass im Falle einer mechanischen Einarbeitung von biologisch aktiven Substanzen in die Vliesstoffe, insbesondere wenn die Substanzen zusammen mit einem Faserrohmaterial zu einem Vliesstoff verarbeitet werden, sowohl die Fasern als auch die biologisch aktiven Substanzen geschädigt werden können.

Die Schädigung erfolgt vor allem durch Hitzebehandlung bei der Herstellung der Fasern. Daher sind einige Verfahren für die Herstellung von Vliesstoffen aus thermoinstabilen Substanzen wie Polysacchariden und Polypeptiden und für die Einarbeitung von thermolabilen biologischen Substanzen wie Enzymen oder Zellen nicht geeignet.

Die aus der EP 2 042 199 A2 bekannten Vliesstoffe überwinden zumindest einen Teil der vorhergehend genannten Nachteile. Die Fasern der Vliesstoffe sind dabei mit biologisch aktiven Substanzen ausgerüstet, die in den Fasern verteilt sind. Die Fasern bestehen aus einem Faserrohmaterial, das natürliche Polymere aufweist.

Dabei können die biologisch aktiven Substanzen während des
Herstellungsprozesses der Vliesstoffe mittels eines Rotationsspinnverfahrens in den Fasern verteilt werden, wobei das Verfahren derart schonend durchgeführt werden kann, dass sowohl die Fasern bzw. das Faserrohmaterial, als auch die biologisch aktiven Substanzen nicht geschädigt werden.

Vliesstoffe, die natürliche Polymere aufweisen oder daraus bestehen, weisen häufig eine geringe mechanische Stabilität auf, die sowohl bei der Lagerung als auch bei der Anwendung nachteilig ist. Zudem ist der Einsatz natürlicher Polymere als Faserrohmaterial kostenintensiv.

Demzufolge ist der Anwendungsbereich bzgl. der vorhergehend genannten Vliesstoffe eingeschränkt. Somit besteht ein zunehmender Bedarf an Vliesstoffen, die zumindest gegenüber Vliesstoffen mit natürlichen Polymeren eine höhere mechanische Stabilität aufweisen.

### Darstellung der Erfindung

Der vorliegenden Erfindung liegt die Aufgabe zugrunde, einen bioabbaubaren Vliesstoff anzugeben, die sich durch eine höhere mechanische Stabilität und durch eine kostengünstigere Herstellung auszeichnet.

Erfindungsgemäß wird diese Aufgabe durch die Merkmale des Patentanspruchs 1 gelöst.

Erfindungsgemäß ist zunächst erkannt worden, dass Faserrohmaterialien aus Polymeren, die eine ausreichende Verfestigung erlauben, häufig nicht wundverträglich, insbesondere nicht bioresorbierbar sind. Überraschenderweise wurde festgestellt, dass bei Verwendung von Faserrohmaterialien aus synthetischen Polymeren eine höhere mechanische Stabilität des Vliesstoffs erreicht werden kann, ohne auf eine biologische Abbaubarkeit oder Biokompatibilität zu verzichten. Dabei ist insbesondere erkannt worden, dass natürliche Polymere für bestimmte physiologische Anwendungen aufgrund mangelnder mechanischer Eigenschaftenim Gegensatz zu synthetischen Polymeren ungeeignet sind. Überdies ist erkannt worden, dass die Verwendung von synthetischen Polymeren kostengünstiger ist als die Verwendung natürlicher. Der Gegenstand der Erfindung ist daher ein Vliesstoff, umfassend Fasern aus einem Faserrohmaterial, wobei die Fasern zumindest eine biologisch aktive Substanz aufweisen, die zumindest teilweise in und/ oder auf den Fasern angeordnet ist. Das Faserrohmaterial weist als polymeren Bestandteil zumindest ein synthetisches Polymer in unmodifizierter und/ oder modifizierter Form auf, wobei das synthetische Polymer biologisch abbaubar und/ oder biokompatibel ist. Hierdurch sind kostengünstige und mechanisch stabile Vliesstoffe mit guter biologischer Abbaubarkeit bzw. Biokompatibilität angegeben.

Folglich ist die eingangs genannte Aufgabe gelöst.

Im Sinne der Erfindung bedeutet "biologisch aktive Substanz", dass die Substanz gezielt biochemisch, chemisch und/oder physikalisch auf biologische Prozesse einwirkt, wie beispielsweise das Wachstum von Bakterien oder Keimen oder die Wundheilung. Bevorzugt werden durch biologisch aktive Substanzen die biologischen Prozesse zumindest teilweise gehemmt, begünstigt oder beschleunigt.

Durch die mechanische Stabilität des Vliesstoffs ist dieser sowohl bei der Lagerung als auch bei der Anwendung einer geringeren Zersetzungsgefahr ausgesetzt. Somit ist ein derartiger Vliesstoff prädestiniert für den Einsatz in oder an Organismen.

Der Vliesstoff könnte zumindest ein synthetisches Polymer aus folgender Gruppe aufweisen: Polylactid (PLA), Polyglycolid (PGA), Polycaprolacton (PCL), Polyhydroxybuttersäure (PHB), Polydioxanonsäure (PDS), Polyvinylpyrrolidon (PVP), Polyesteramid (PEA), Polyanhydrid, Polyorthoester, Polyphosphoester, Polyvinylalkohol (PVA), Polyethylenoxid (PEO), Polyethylenglycol (PEG), Polyethylen (PE) oder Polypropylen (PP), Siliziumverbindungen (z.B. SiO₂) bzw. Copolymere dieser Stoffe, wobei zumindest ein synthetisches Polymer in unmodifizierter und/ oder modifizierter Form vorliegt.

Ein synthetisches Polymer ist ein Polymer, dass aus synthetisierten Monomeren aufgebaut ist. Dabei versteht man unter Synthese das Umsetzen von einer oder mehreren Verbindungen zu einer neuen Verbindung. Das synthetische Polymer kann auch aus fossilen Rohmaterialien hergestellt sein. Synthetische Polymere weisen im Vergleich zu z.B. natürlichen Polymeren eine höhere Biostabilität auf, da sie Strukturen aufweisen, die in der Natur weniger bekannt sind und somit auch durch Organismen und/ oder deren Produkte weniger stark angegriffen werden können. Des Weiteren können auch modifizierte synthetische Polymere verwendet werden. Dabei versteht man unter Modifizierung eine Synthese, bei der die grundlegende Struktur der Verbindung, insbesondere das Kohlenstoffgerüst, beibehalten wird und funktionelle Gruppen eingeführt werden. So kann beispielsweise durch Einführen von Hydroxygruppen eine stärkere Hydrophilie des synthetischen Polymers erreicht werden. Somit modifiziert man das Polymer hinsichtlich seiner hydrophilen Eigenschaften, ohne die kennzeichnende Grundstruktur des Polymers zu ändern.

So können als synthetisches Polymer Polylactid (PLA), Polyglycolid (PGA), Polycaprolacton (PCL), Polyhydroxybuttersäure (PHB), Polydioxanonsäure (PDS), Polyvinylpyrrolidon (PVP), Polyesteramid (PEA), Polyanhydrid, Polyorthoester, Polyphosphoester, Polyvinylalkohol (PVA), Polyethylenoxid (PEO), Polyethylenglycol (PEG), Polyethylen (PE) oder Polypropylen (PP), Siliziumverbindungen (SiO₂ bzw. Copolymere dieser Stoffe eingesetzt werden.

Bevorzugt wird Polylactid (PLA), Polyglycolid (PGA), Polycaprolacton (PCL) und Polyethylenoxid (PEO), Polyethylenglycol (PEG), bzw. deren Copolymere, besonders bevorzugt werden Milchsäure-basierte Polymere wie z.B. Polylactid (PLA) und Polyethylenoxid (PEO) bzw. Polyethylenglycol (PEG) und ganz besonders bevorzugt wird Polylactid (PLA) und Poly(lactid-co-glycolid) verwendet.

Das synthetische Polymer könnte aufgrund von funktionellen Gruppen selbst eine biologisch aktive Substanz darstellen. Dadurch kann das synthetische Polymer aufgrund von z.B. funktionellen Gruppen selbst eine bioaktive Wirkung entfalten.

Das Polymer könnte mit biologisch aktiven Molekülen wie zum Beispiel adhäsionsfördemden Peptidsequenzen (z.B. RGD, IKVAV etc.), Wachstumsfaktoren (BMP, EGF, PDGF, etc.), proteinadsorptionsvermeidenden Substanzen (z.B. Polyethylenoxide) oder antimikrobiell aktiven Substanzen (alkylsubstituierten quartären Aminogruppen) modifiziert sein. Durch Abbau des Polymeres können die biologisch aktiven Substanzen aus den Polymerkojugaten kontrolliert freigesetzt werden.

Bei den hier beschriebenen Vliesstoffen handelt es sich vorzugsweise um nicht gewebte ("non woven") Materialien. Diese sind bevorzugt nicht nachverdichtet.

Die Fasern könnten miteinander verdrillt sein und/oder eine verdrillte Struktur aufweisen. Einige Fasern, bevorzugt mehr als 5 %, besonders bevorzugt mehr als 10 % und ganz besonders bevorzugt mehr als 20 % der Fasern, können miteinander verdrillt sein oder eine verdrillte Struktur aufweisen. Die Verdrillungen stellen sich überraschend während des Rotationsspinnens ein und begünstigen zusätzlich die Festigkeit des Vliesstoffs.

Zumindest ein Teil der Fasern könnte als Nanofasem ausgebildet sein. Ein Vliesstoff dieser Ausgestaltung kann besonders leicht und dünn ausgestaltet sein.

Die biologisch aktive Substanz könnte zumindest teilweise als Beschichtung, Imprägnierung oder als Verkapselung vorliegen. Die biologisch aktive Substanz kann als Imprägnierung, an der Oberfläche angereichert, als Verkapselung z.B. in Art von Nanoemulsionen, als Dispersion oder als molekular verteilter Wirkstoff in bzw. auf der Faser vorliegen. Die biologisch aktive Substanz kann sowohl simultan als auch in einem weiteren Verfahrensschritt eingebracht werden.

Die zumindest eine biologisch aktive Substanz könnte in den Fasern verteilt bzw. in und/ oder auf den Fasern angeordnet sein. Hierdurch lässt sich eine allmähliche Abgabe der biologisch aktiven Substanz mit lang anhaltender Wirkung einstellen. Dabei schließt die Verteilung "in den Fasern" nicht aus, dass die Substanz zusätzlich auf den Fasern, also an deren Oberfläche gebunden, vorliegt. Bevorzugt ist die Verteilung in den Fasern homogen. "Homogen" bedeutet dabei, dass die aktive Substanz im Gegensatz zu Vliesen oder Vliesstoffen, die mit Wirkstoffen nachbehandelt werden, innerhalb der Fasern im wesentlichen gleichmäßig eingeschlossen ist und nicht ungleichmäßig verteilt ist, beispielsweise aufgrund einer unvollständigen Diffusion in die Fasern. Dem steht nicht entgegen, dass sich möglicherweise erhöhte Mengen der Wirkstoffe an der Faseroberfläche befinden, beispielsweise aufgrund von Grenzflächeneffekten.

Die biologisch aktive Substanz kann demzufolge zusätzlich auf den Fasern verteilt sein, um eine rasche Abgabe an den menschlichen Körper sicher zu stellen. Dies kann durch ein Besprühen des Vliesstoffs mit der Substanz oder ein Tränken in der Substanz ermöglicht werden.

Zumindest eine biologisch aktive Substanz könnte nanoskalig vorliegen. Die biologisch aktive Substanz kann in den Fasern nanoskalig vorliegen. Unter nanoskaligen Strukturen versteht man Bereiche jeglicher Morphologie, die zumindest in einer Raumrichtung Dimensionen im Nanometerbereich aufweisen. Hierdurch erlangt die biologisch aktive Substanz eine hohe Mobilität. Eine nanoskalig vorliegende biologisch aktive Substanz zeigt eine besonders hohe Reaktivität, wenn sie mit Bakterien, Viren, Pilzen oder Sporen in Kontakt gebracht wird. Des Weiteren gibt der Vliesstoff den wirkenden Stoff sehr leicht an Medien ab, die mit diesem in Kontakt treten. Insoweit zeichnet sich der Vliesstoff durch eine hohe Abgabefähigkeit in Bezug auf die biologisch aktive Substanz aus.

Zumindest eine biologisch aktive Substanz könnte aus folgender Gruppe ausgewählt sein: ein Antiseptikum, ein antimikrobiell wirksamer Stoff, ein Antibiotikum, ein Medikament, ein Wachstumsfaktor, ein Enzym und/ oder eine Zelle, ein biologisch aktives Metall oder eine biologisch aktive Metallegierung. Als "antiseptisch" bezeichnet man eine Wirkung, die zur Verminderung der Anzahl von infektiösen Keimen und damit zur Verhinderung einer Infektion führt. Als "Substanz" im Sinne der Verbindungen werden nicht nur einzelne chemische Verbindungen verstanden, sondern auch Substanzgemische oder komplexe Einheiten wie Zellen oder Extrakte.

Die Fasern und/ oder zumindest eine biologisch aktive Substanz könnte oberhalb von 200°C instabil sein. Das Faserrohmaterial, die Fasern und/ oder die biologisch aktive Substanz können oberhalb einer vorbestimmten Temperatur instabil sein. "Instabil" bedeutet, dass diese Materialien sich innerhalb einer vorbestimmten Zeit bei dieser Temperatur so verändern, dass die Verwendbarkeit zur Erzeugung eines Vliesstoffes oder die biologische Aktivität signifikant abnehmen. "Signifikant" bedeutet dabei beispielsweise, dass die biologische Aktivität um mindestens 10 % abnimmt. Beispielsweise neigen Enzyme bei solchen Temperaturen zum Denaturieren, während sich instabile Antibiotika chemisch zersetzen. Fasermaterialien wie Polysaccharide, Proteine, Polypeptide oder Gelatine neigen bei erhöhten Temperaturen zum Denaturieren oder Verklumpen.

Der Vliesstoff könnte eine Reißfestigkeit bei einem spezifischen Flächengewicht von 140 bis 180 g/m² im trockenen Zustand von mindestens 0,10 N/mm² und/ oder eine Reißdehnung in hydratisiertem Zustand von mindestens 100% aufweisen. Der Vliesstoff kann eine Reißfestigkeit und/ oder eine Reißdehnung aufweisen. Bevorzugt weist er eine Reißfestigkeit bei einem spezifischen Flächengewicht von 140 bis 180 g/m² im trockenen Zustand von mindestens 0,1 N/mm², besonders bevorzugt von mindestens 0,2 N/mm² und ganz besonders bevorzugt von mindestens 0,3 N/mm² auf. Alternativ oder zusätzlich weist er bevorzugt eine Reißdehnung in hydratisiertem Zustand von mindestens 100 %, besonders bevorzugt von mindestens 200 % und ganz besonders bevorzugt von mindestens 300 % auf, Ein solcher Vliesstoff kann besonders vorteilhaft als Implantat für Hartgewebe, insbesondere für Knochen oder Knorpel, verwendet werden, da er mechanisch beanspruchbar ist.

Der Vliesstoff könnte eine offene Porenstruktur mit einer Luftdurchlässigkeit von mindestens 0,5 I/min*cm² aufweisen. Der Vliesstoff kann eine offene Porenstruktur mit einer Luftdurchlässigkeit aufweisen, die gemäß DIN 9237 ermittelt wird. Bevorzugt weist der Vliesstoff eine Luftdurchlässigkeit von mindestens 0,5 l/min*cm², besonders bevorzugt von mindestens 0,6 l/min*cm², und ganz besonders bevorzugt von mindestens 0,7 l/min*cm² auf. In eine solche Porenstruktur können Zellen besonders gut einwandern bzw. einwachsen.

Der antimikrobiell wirksame Stoff kann zumindest ein Nebengruppenelement, ein Metall, besonders bevorzugt ein Edelmetall, oder eine Metallegierung umfassen. Nebengruppenelemente zeichnen sich durch antimikrobielle Wirkung aus. Vor diesem Hintergrund ist denkbar, dass mehrere Nebengruppenelemente gemeinsam vorliegen, um unterschiedlichen Bakterienarten selektiv zu begegnen. Es hat sich in Versuchsreihen gezeigt, dass sich in Bezug auf die antimikrobielle Wirksamkeit eine Rangfolge der verwendeten Stoffe ergibt. Diese lässt sich wie folgt darstellen. Silber ist der wirksamste Stoff, gefolgt von Quecksilber, Kupfer, Cadmium, Chrom, Blei, Kobalt, Gold, Zink, Eisen und schließlich Mangan. Vor diesem Hintergrund ist denkbar, auch Hauptgruppenelemente zu verwenden, welche eine antimikrobielle Wirkung zeigen. Der antimikrobiell wirkende Stoff kann eine Gold-Silber Mischung umfassen oder ausschließlich aus einer Gold-Silber Mischung bestehen. Mischungen dieser Art zeigen eine besonders hohe antimikrobielle Wirksamkeit. Es hat sich überraschenderweise gezeigt, dass die Anwesenheit von Gold die antimikrobielle Wirkung von Silber noch steigert. Der antimikrobiell wirksame Stoff kann auch Silber enthalten. Silber ist besonders als antimikrobiell wirkender Stoff geeignet, da es für Menschen nahezu ungiftig ist. Des Weiteren zeigt Silber ein relativ geringes allergenes Potential. Silber wirkt als antiseptische Substanz in geringen Konzentrationen über einen langen Zeitraum auf eine Vielzahl von Infektionskeimen. Die meisten bekannten Bakterien zeigen darüber hinaus keine Resistenz gegen Silber.

Des Weiteren kann die biologisch aktive Substanz in Kombination mit geeigneten Trägerstoffen, Stabilisatoren oder sonstigen galenischen Zusätzen in das Vlies oder in den Vliesstoff eingearbeitet werden. Bei den Stabilisatoren kann es sich unter anderem um etablierte Substanzen wie Stärke, Gelatine, Chitosan, Polyvinylpyrrolidon oder Polyethylenoxid handeln.

Die Faserrohmaterialien können bevorzugt ausgewählt werden aus der Gruppe bestehend aus synthetischen Polymeren und Polymeren aus fossilen Rohmaterialien, jeweils in unmodifizierter und/ oder modifizierter Form.

Ein Medizinprodukt, Arzneimittel, Wundverband, Wattetupfer oder Implantat könnte einen Vliesstoff der hier beschriebenen Art enthalten.

Der hier beschriebene Vliesstoff könnte zur Herstellung eines Arzneimittels für die Wundheilung verwendet werden. Vliesstoffe der hier beschriebenen Art können im medizinischen Bereich Verwendung finden, da sie im Hinblick auf ihre Gewebestruktur und stoffliche Zusammensetzung sehr gut modifizierbar sind. Beispielsweise ist denkbar, die Gewebestruktur so einzustellen, dass sie als Scaffold oder Implantat fungieren können, wenn nämlich die Fasergewebestruktur mit dem menschlichen Gewebe gut verwachsen kann. Weitere medizinische Anwendungen wie die Verwendung als Zellwachstumsträger sind denkbar.

Insbesondere könnte ein Medizinprodukt, Wattetupfer oder Implantat einen Vliesstoff der hier beschriebenen Art enthalten.

Der hier beschriebene Vliesstoff kann auch als Implantat oder als Teil eines Implantats verwendet werden. Dabei ist die Verwendung in bioresorbierbaren oder langzeitstabilen Implantaten möglich. Beispielsweise können die Implantate bei chirurgischen Eingriffen eingesetzt werden und durch die biologischen aktiven Substanzen gezielt therapeutisch wirken.

Der hier beschriebene Vliesstoff eignet sich besonders zur Herstellung eines Wattetupfers oder Tupfers, da er hinreichend stabil ist und desinfizierend wirkt.

Weitere Ausrüstungen des Vliesstoffs sind möglich. Der Vliesstoff könnte mit rekombinanten Wachstumsfaktoren, autologen Wachstumsfaktoren, insbesondere Thrombozyten-Präparationen, Adhäsionsfaktoren, insbesondere RDG-Peptiden, und/ oder autologen Zellpräparationen, insbesondere Knochenmarkaspiraten, ausgerüstet sein.

Zur Herstellung eines hier beschriebenen Vliesstoffs könnte ein Rotationsspinnverfahren verwendet werden, bei dem das Faserrohmaterial in ein Behältnis gegeben wird, wobei das Behältnis in Rotation versetzt wird und wobei das fluidisierte Faserrohmaterial durch Zentripetalkräfte aus dem Behältnis in Form von Fasern ausgetragen wird.

Überraschenderweise erlaubt diese Verfahren die gleichmäßige Einarbeitung biologisch aktiver Substanzen in Vliesstoffe während der Erzeugung der Vliese in einem Rotationsspinnverfahren. Da bei dem Rotationsspinnverfahren die Faserrohmaterialien und die biologisch aktiven Substanzen sehr kurze Verweilzeiten in der Spinnvorrichtung aufweisen, die beispielsweise im Bereich von Mikrosekunden liegen können, werden weder die Faserrohmateriatien noch die aktiven Substanzen geschädigt. Auf diese Art werden neue Vliese oder Vliesstoffe erhalten, die nach bekannten Verfahren nicht herstellbar waren. Bei den bekannten Elekfirospinnvertahren sind Verweildauern von mehreren Minuten üblich.

Des Weiteren konnte beobachtet werden, dass synthetische Polymere sich überraschend gut zu einem Vlies ablegen lassen, wobei sich gleichzeitig mittels des Herstellungsverfahrens in die Fasern biologische Wirkstoffe einarbeiten lassen. Weiterhin wurde erkannt, dass die Fasern des Vlieses zum Teil ohne Phasengrenze ineinander übergehen, miteinander vernetzen und dadurch einen festen Verbund ausbilden. Der entstehende Vliesstoff zeigt ohne weitere Verfestigungsmaßnahmen überraschend eine hinreichend hohe Reißfestigkeit, um als Verbandsmaterial oder Wundauflage Verwendung zu finden. Die biologisch aktive Substanz wird in ihrer Wirkung nicht durch Verfestigungsmaßnahmen negativ beeinträchtigt. Weiter konnte festgestellt werden, dass mittels eines Rotationsspinnverfahrens der Durchmesser der Fasern in einer engen Verteilung einstellbar ist. Durch das Rotationsspinnverfahren sind Fasern mit einem Durchmesser von im Mittel 0,3 bis 500 um, im Mittel 3 bis 200 µm und sogar im Mittel 5 bis 100 µm erzeugbar. Die enge Verteilung des Durchmessers der Fasern sowie die Steuerung der Restfeuchte erlauben einen homogenen und stabilen Aufbau des Vliesstoffs ohne teure zusätzliche Verfestigungsmaßnahmen.

Die austretenden Fasern könnten gerichtet, kontaktlos geführt werden. Eine kontaktlose und definierte Führung der Fasern, bevor diese auf einer Ablageeinrichtung auftreffen, erlaubt eine Modifikation der Fasern. So kann allein die Dauer der Führung bzw. die Richtung der Führung Einfluss auf die Faserlänge, den Faserdurchmesser sowie die Faserstruktur nehmen. Eine gerichtete kontaktlose Führung erzeugt ein homogeneres Faserspektrum, als ein Herstellungsprozess ohne Führung. Ganz konkret kann allein durch die Führung die Breite einer Verteilungskurve sämtlicher Fasereigenschaften eingestellt werden. Hierdurch kann die Menge an Fasern mit unerwünschter Fasergeometrie erheblich reduziert werden.

In einer konstruktiv besonders günstigen Ausgestaltung könnten die Fasern durch eine Absaugeinrichtung geführt werden. Dabei ist denkbar, dass die Fasern durch einen Gasstrom transportiert werden. Sofern der Gasstrom laminar ausgebildet ist, können die Fasern zwischen den laminaren Schichten gestreckt und geformt werden.

Als Gas kann Luft fungieren. Luft ist ein kostengünstiges Gas, welches sich dadurch auszeichnet, dass ein Herstellungsprozess bei Atmosphärendruck durchgeführt werden könnte. Denkbar ist auch, dass anstelle von Luft weitere Gase, insbesondere Inertgase wie Stickstoff, verwendet werden. Hierdurch ist sicher gestellt, dass das Faserrohmaterial verarbeitbar ist, weiches reaktive Gruppen umfasst oder zu Nachreaktionen nach dem Verlassen des Behältnisses neigt.

Es können Fasern hergestellt werden, die einen Durchmesser von bevorzugt 0,3 bis 500 µm, besonders bevorzugt 0,4 bis 400 µm, ganz besonders bevorzugt 0,5 bis 300 µm aufweisen. Insoweit ist eine Herstellung von Nanofasem möglich, ohne dass ein elektrostatisches Feld verwendet werden muss.

Für die Durchführung des Rotationsspinnverfahrens wird besonders bevorzugt eine Vorrichtung oder ein Behältnis verwendet, wie es in der DE 102005048939 A1 beschrieben wird. Auf diese Vorrichtungen und Behältnisse wird hier ausdrücklich Bezug genommen. Eine Vorrichtung gemäß DE 102005048939 A1 umfasst ein Behältnis zur Aufnahme von Faserrohmaterial, wobei das Behältnis durch mindestens eine Wärmequelle erwärmbar ist wobei das Behältnis in Rotation versetzbar ist und wobei das Behältnis Austrittsbereiche für das Faserrohmaterial aufweist, wobei den Austrittsbereichen Mittel zur gerichteten, kontaktlosen Führung des aus dem Behältnis austretenden Faserrohmaterials zugeordnet sind.

Die Austrittsbereiche des Behältnisses können als Durchgänge ausgestaltet sein. Hierbei ist denkbar, dass die Durchgänge kreisförmig, oval oder rechteckförmig ausgestaltet sind. Ganz in Abhängigkeit von der Form der Durchgänge kann auf die Fasergeometrie Einfluss genommen werden.

Die Durchgänge können einen Durchmesser oder eine Weite bis bevorzugt 500 µm, besonders bevorzugt 400 µm, ganz besonders bevorzugt 300 µm aufweisen. Diese Dimensionierung hat sich als besonders vorteilhaft für die Erzeugung von Nanofasem und Mikrofasern erwiesen. Die Durchgänge könnten in einem Abstand von einem Zentimeter voneinander positioniert sein. Denkbar ist auch, dass die Durchgänge in mehreren Reihen übereinander angeordnet sind. Hierdurch ist der Durchsatz an Faserrohmaterial auf besonders einfache Weise steigerbar. Durch Verringerung oder Vergrößerung des Durchmessers ist es möglich, Nano- bzw. Mikrofasern im Bereich von 0,3 bis 500 µm zu erzeugen.

Das Behältnis kann mit bis zu 25.000 Umdrehungen pro Minute rotierbar sein. Durch diese hohe Drehzahl ist es möglich, Nanofasem mit einem Durchmesser von höchstens 100 nm zu erzeugen. Üblicherweise werden Nanofasem durch ein elektrisches Spinnverfahren unter Verwendung eines elektrischen Feldes erzeugt. Durch eine geeignete konstruktive Ausgestaltung des Behältnisses sowie sehr hohe Umdrehungszahlen ist es jedoch möglich, ohne elektrisches Feld Nanofasern zu erzeugen. Durch Wahl der Rotationsgeschwindigkeit und Viskosität des Faserrohmaterials können auch Fasern mit größerem Durchmesser erzeugt werden.

Die durchschnittliche Verweilzeit des Faserrohmaterials kann in dem Behältnis so kurz sein, dass die Faserrohmaterialien und/ oder die biologisch aktiven Substanzen nicht in unerwünschter Weise verändert werden. Daher nimmt die Verwendbarkeit des Fasermaterials oder die biologische Aktivität der Substanz auch bei einer hohen Temperatur nicht signifikant ab. Bevorzugt ist die durchschnittliche Verweilzeit kürzer als 100 oder 10 Sekunden, besonders bevorzugt kürzer als 1 Sekunde oder 100 Mikrosekunden und ganz besonders bevorzugt kürzer als 10 oder 2 Mikrosekunden. Bei derartig kurzen Verweilzeiten können überraschenderweise auch biologisch aktive Substanzen wie Proteine, hitzeinstabile Antibiotika, Wachstumsfaktoren oder Zellen in Fasern eingearbeitet werden, insbesondere auch in hitzeempfindliche Faserrohmaterialien. In einer besonders bevorzugten Ausführungsform werden hitzeinstabile Substanzen in hitzeinstabile Fasern eingearbeitet. Es wurde festgestellt, dass sich die in DE 102005048939 A1 beschriebenen Vorrichtungen so einstellen lassen, dass die bevorzugten kurzen Verweilzeiten erzielt werden können.

Das Behältnis kann auf 300° C erwärmbar sein. Diese Ausgestaltung ermöglicht vorteilhaft eine Verwendung nahezu aller thermoplastischen faserbildenden Werkstoffe als Faserrohmaterial. Hierbei ist denkbar, dass Polyester, Polyamide, Polyurethane, Polylactide und Polyhydroxybutyrat/ Polyhydroxyvalerat-Copolymere sowie native Zucker, z.B. Saccharose, oder Mischungen aus den genannten Stoffen verwendet werden. Darüber hinaus könnte das Faserrohmaterial Polyolefine oder reaktive Polymere umfassen. Denkbar ist hierbei, dass Polypropylen, mit Acrylsäure gepfropftes Polypropylen und/ oder modifiziertes Polypropylen verwendet werden. Die Verwendung von biologisch abbaubaren Stoffen wie Gelatine als Faserrohmaterial ermöglicht die Erzeugung von Fasern, die problemlos entsorgbar sind. Des Weiteren können mit diesen Fasern medizinische Produkte wie Wundverbände oder Zellwachstumsträger gefertigt werden. Alle genannten Stoffe können allein oder in Mischung mit anderen als Faserrohmaterial Verwendung finden.

Dem rotierenden Behältnis kann eine Ablageeinrichtung zur Aufnahme von Faserrohmaterial zugeordnet sein. Die Ablegeeinrichtung kann als Plattform ausgestaltet sein, auf der die Fasern zur Bildung eines Faserflors oder Vlieses abgelegt werden können. Denkbar ist auch, dass die Ablageeinrichtung eine rotierende Einrichtung ist, auf welcher Fasern zur Beschichtung eines zylindrischen Körpers oder zur Erzeugung eines Wickelvlieses aufgenommen werden.

Zwischen der Ablageeinrichtung und dem Behältnis kann eine elektrische Potentialdifferenz bestehen. Diese konkrete Ausgestaltung erlaubt die Fertigung von elektrostatisch aufgeladenen Fasern.

Des Weiteren ist denkbar, dass die elektrische Potentialdifferenz zur Unterstützung der Herstellung von Nanofasem verwendet wird. Hierbei addieren sich die Effekte der Zentripetalkräfte und des elektrischen Feldes, d.h. das Faserrohmaterial wird einerseits durch die Zentripetalkräfte in dünnen Fäden tangential vom rotierenden Behältnis weg geschleudert und des Weiteren durch das elektrische Feld ggf. noch weiter aufgesplittet. Insoweit ist denkbar, einen Fertigungsprozess zu realisieren, durch welchen Fasern im Nanometerbereich herstellbar sind.

Das Faserrohmaterial kann bereits in fluidisierter Form in das Behältnis eingebracht werden. Hierdurch ist es möglich, einen kontinuierlichen Prozess durchzuführen, indem nämlich das Faserrohmaterial außerhalb des Behältnisses erwärmt wird. Denkbar ist jedoch auch, das Faserrohmaterial erst zu fluidisieren, nachdem es in das Behältnis eingebracht wurde.

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche, andererseits auf die nachfolgende Erläuterung bevorzugter Ausführungsbeispiele des erfindungsgemäßen Vliesstoffs anhand der Zeichnung zu verweisen.

In Verbindung mit der Erläuterung der bevorzugten Ausführungsbeispiele anhand der Zeichnung werden auch im Allgemeinen bevorzugte Ausgestaltungen und Weiterbildungen der Lehre erläutert.

### Kurzbeschreibung der Zeichnung

In der Zeichnung zeigen
- Fig. 1: eine Lichtmikroskop-Aufnahme eines beschriebenen Vliesstoffs gemäß Ausführungsbeispiel 1,
- Fig. 2: eine Lichtmikroskop-Aufnahme eines beschriebenen Vliesstoffs gemäß Ausführungsbeispiel 2 und
- Fig. 3: eine Lichtmikroskop-Aufnahme eines beschriebenen Vliesstoffs gemäß Ausführungsbeispiel 3.

### Ausführung der Erfindung

Ausführungsbeispiel 1:
Ein Vliesstoff mit Silber als antimikrobiellem Stoff wird durch ein Rotationsspinnverfahren wie folgt hergestellt:
   Zunächst wird eine 25%ige Polyvinylpyrrolidon-Lösung hergestellt. Zur Verwendung kommt ein Polyvinylpyrrolidon des Typs Kollidon 90 F der Firma BASF AG. Das Polyvinylpyrrolidon wird in Wasser mit Hilfe eines Magnetrührers gelöst, auf 60°C erhitzt und eine Stunde lang mit Ultraschall behandelt (IKA, Germany). Der Polyvinylpyrrolidon-Lösung werden 1000 ppm nanoskalige Silberpartikel zugegeben. Es wurden Silberpartikel der Firma Aldrich (Nanopulver <100 nm Partikelgröße) verwendet. Diese Mischung wird weitere 60 Minuten mit dem Ultraschallbad bei 60° C homogenisiert. Hierdurch entsteht eine Endkonzentration von 40 mg Silber /(kg Polyvinylpyrrolidon-Lösung). Die Partikel des Silbers können in der Polyvinylpyrrolidon-Lösung Agglomerate bilden, die durch Rühren der Polyvinylpyrrolidon-Lösung aufgelöst werden.

Die Polyvinylpyrrolidon-Lösung wird bei 60° C mittels einer Schlauchpumpe als Faserrohmaterial in das Behältnis einer Vorrichtung zum Rotationsspinnen gemäß der DE 102005048939 A1 geführt.

Das Behältnis hat eine Temperatur von ca. 70° C und rotiert mit einer Drehzahl von 4500 U/min. Im Behältnis befinden sich Ausnehmungen, die als Löcher mit einem Durchmesser von 0,3 mm ausgestaltet sind. Durch die Zentripetalkraft wird das Fasenrohmaterial durch die Ausnehmungen gepresst und zu Fasern gesponnen, die durch eine Absaugeinrichtung verstreckt werden. Die Absaugeinrichtung befindet sich unterhalb des Behältnisses.

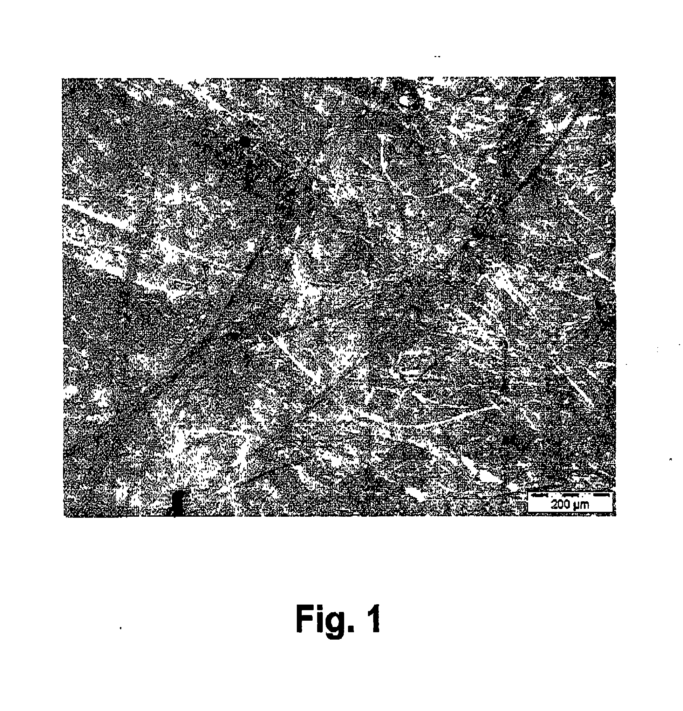

Es wird ein antimikrobiell wirksamer, bioresorbierbarer Vliesstoff aus Polyvinylpyrrolidon erhalten. Der Vliesstoff aus Polyvinylpyrrolidon wird entweder während des Spinnprozesses oder anschliessend vernetzt. Eine anschliessende Vernetzung kann z,B. über eine Strahlenvemetzung (GammaStrahlung oder Elektronenbestrahlung) unter Verwendung sterilisationsüblicher Dosen (25 kGy) erfolgen. Andere Möglichkeiten zur Vernetzung sind z.B. die Zugabe von 250 mg Irgacure 2022 und Bestrahlung mit einer 500 W UV-Lampe über einen Zeitraum von mehreren Stunden oder die Zugabe von einem Milliliter 32%iger HCl zur Spinnlösung und anschliessende Behandlung mit Formaldehyd in der Gasphase. Ein auf diese Weise behandelter Vliesstoff aus Polyvinylpyrrolidon ist über einen längeren Zeitraum (> 2 Stunden) wasserstabil. Gemäß einer Charakterisierung nach ICP (gemäß EN ISO 11885) beträgt die Silberkonzentration im Vliesstoff 32 mg/kg. Fig. 1 zeigt eine Mikroskopaufnahme des hier beschriebenen Vliesstoffs.

Ausführungsbeispiel 2:
Ein Vliesstoff mit minimierter unspezifischer Proteinadsorption wird durch ein Rotationsspinnverfahren wie folgt hergestellt:
   Zunächst wird eine 5%ige Hexakis(undeka(ethylenglycol))-succinimidylester/2-amino-N1,N5-bis(15,20-dioxo-2,5,8,11-tetraoxa-14,19-diazahenicosan-21-yl)-N1, N5-di(2,5,8,11-tetraoxatridecan-13-yl)pentandiamid-Lösung hergestellt. Hierzu werden Hexakis(undeka(ethylenglycol))-succinimidylester des Typs PEG2060 mit einem Molekulargewicht (M_{w}) von 4215,07 g/mol und 2-amino-N1,N5-bis(15,20-dioxo-2,5,8,11-tetraoxa-14,19-diazahenicosan-21-yl)-N1,N5-di(2,5,8,11-tetraoxatridecan-13-yl)pentandiamid des Typs PEG0173 mit einem Molekulargewicht (M_{W}) von 1190 g/mol (beide von der Fima IRIS Biotech GmbH) in Aceton gelöst (Massenverhältnis 10/1).

Zu dieser Lösung werden 25 Gewichtsprozent Poly(D,L-lactid-co-glycolid) gegeben. Zur Verwendung kommt ein Poiy(D,L-lactid-co-glycolid) 50:50 des Typs Resomer RG 504 der Firma Boehringer Ingelheim GmbH & Co. KG. Die Poly(D,L-lactid-co-glycolid)-Lösung wird mit Hilfe eines Ultra-Turrax (IKA, Germany) mit 22000 Umdrehungen für 60 Sekunden homogenisiert.

Die Pol(D,L-lactid-co-glycolid) /Hexakis(undeka(ethlenglycol))/2-amino-N1,N5-bis(15,20-dioxo-2,5,8,11-tetraoxa-14,19-diazahenicosan-21-yl)-N1,N5-di(2,5,8,11-tetraoxatridecan-13-yl)pentandiamid-succinimidylester-Lösung wird bei Raumtemperatur (23°C) mittels einer Schlauchpumpe als Faserrohmaterial in das Behältnis einer Vorrichtung zum Rotationsspinnen gemäß der DE 102005048939 A1 geführt.

Das Behältnis hat eine Temperatur von ca. 70°C und rotiert mit einer Drehzahl von 4500 U/min, Im Behältnis befinden sich Ausnehmungen, die als Löcher mit einem Durchmesser von 0,3 mm ausgestaltet sind. Durch die Zentripetalkraft wird das Faserrohmaterial durch die Ausnehmungen gepresst und zu Fasern gesponnen, die durch eine Absaugeinrichtung verstreckt werden. Die Absaugeinrichtung befindet sich unterhalb des Behältnisses.

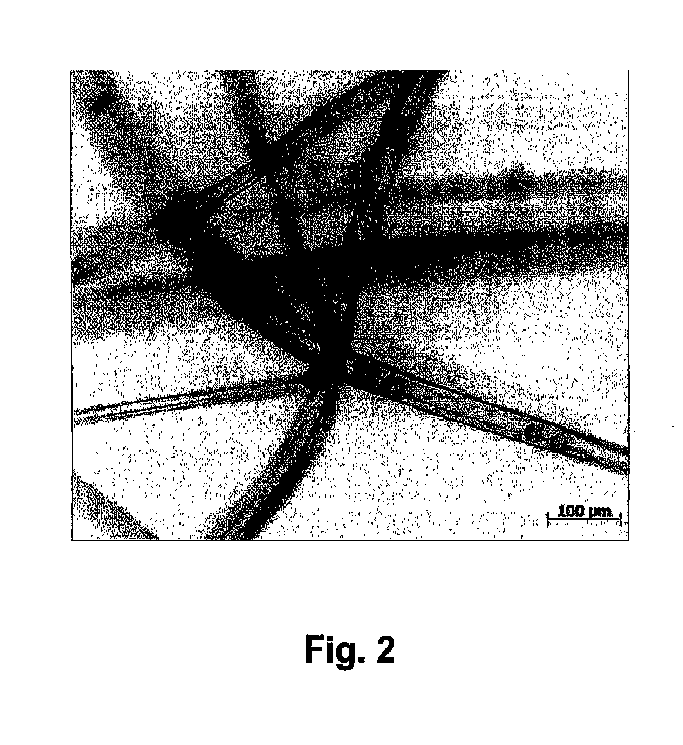

Der Vliesstoff wurde mit einem Lichtmikroskop charakterisiert Flg. 2 zeigt eine Mikroskopaufnahme des hier beschriebenen Vliesstoffs. Die minimierte Proteinadsorption des Vliesstoffes wurde wie in D. Grafahrend, J. Lleixa Calvet, J. Salber, P. D. Dalton, D. Klee, M. Möller, "Control of hydrophilicity and protein adsorption on biofunctionalizable nanofiber scaffolds for tissue engineering", Biotech Bioeng, 2008,101 (3), 609-621 beschrieben, mit Hilfe von zwei Modellproteinen gezeigt.

Ausführungsbeispiel 3:
Ein Vliesstoff mit minimierter unspeziflscher Proteinadsorption und immobilisierten Peptidsequenzen zur Adhäsionsförderung von Fibroblasten wird durch ein Rotationsspinnverfahren wie folgt hergestellt:
   Zunächst wird eine 5%ige Hexakis(undeka(ethylenglycol))-succinimidylester/2-amino-N1,N5-bis(15,20-dioxo-2,5,8,11-tetraoxa-14,19-diazahenicosan-21-yl)-N1,N5-di(2,5,8,11-tetraoxatridecan-13-yl)pentandiamid-Lösung hergestellt. Hierzu werden Hexakis(undeka(ethylengtycol))-succinimidyl ester des Typs PEG2060 mit einem Molekulargewicht (M_{w}) von 4215,07 g/mol und 2-amino-N1,N5-bis(15,20-dioxo-2,5,8.11-tetraoxa-14,19-diazaheicosan-21-yl)-N1,N5-di(2,5,8,11-tetraoxatridecan-13-yl)pentandiamid des Typs PEG0173 mit einem Molekulargewicht (M_{W}) von 1190 g/mol (beide von der Fima IRIS Biotech GmbH) in Aceton gelöst (Massenverhältnis 10/1).

Zu dieser Lösung werden 25 Gewichtsprozent Poly(D,L-lactid-co-glycolid) gegeben. Zur Verwendung kommt ein Poly(D,L-lactid-co-glycolid) 50:50 des Typs Resomer RG 504 der Firma Boehringer Ingelheim GmbH & Co. KG. Die Poly(D,L-lactid-co-glycolid)-Lösung wird mit Hilfe eines Ultra-Turrax (IKA, Germany) mit 22000 Umdrehungen für 60 Sekunden homogenisiert.

Zu dieser Lösung wird ein Äquivalent (bezogen auf

Hexakis(undeka(ethylenglycol))-succinimidylester) der Peptidsequenz RGD (Peptid aus den drei Aminosäuren Arginin, Glycin und Asparaginsäure, kurz Arg-Gly-Asp, des Typs H-1830 von der Firma Bachem) gelöst in 0,2 ml Dimethylsulfoxid und 30 µl Wasser zugegeben und erneut mit Hilfe des Ultra-Turrax homogenisiert.

Die Poly(D,L-lactid-co-glycolid)/Hexakis(undeka(ethylenglycol))/2-amin-N1,N5-bis(15,20-dioxo-2,5,8,11-tetraoxa-14,19-diazahenicosan-21-yl)-N1,N5-di(2,5,8,11-tetraoxatndecan-13-yl)pentandiamidsuccinimidylester/RGD-Lösung wird bei Raumtemperatur (23°C) mittels einer Schlauchpumpe als Faserrohmaterial in das Behältnis einer Vorrichtung zum Rotationsspinnen gemäß der DE 102005048939 A1 geführt.

Das Behältnis hat eine Temperatur von ca. 70° C und rotiert mit einer Drehzahl von 4500 U/min. Im Behältnis befinden sich Ausnehmungen, die als Löcher mit einem Durchmesser von 0,3 mm ausgestaltet sind. Durch die Zentripetalkraft wird das Faserrohmaterial durch die Ausnehmungen gepresst und zu Fasern gesponnen, die durch eine Absaugeinrichtung verstreckt werden. Die Absaugeinrichtung befindet sich unterhalb des Behältnisses.

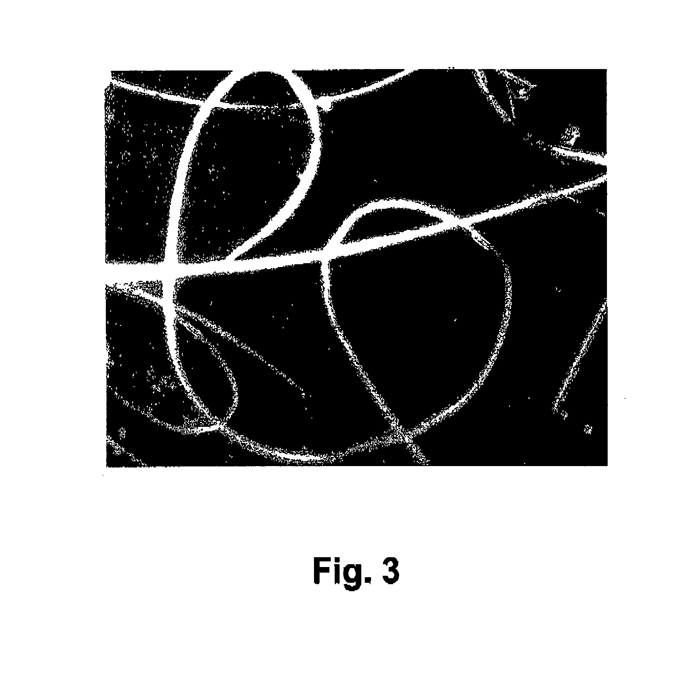

Der Vliesstoff wurde mit einem Lichtmikroskop charakterisiert. Die Zellversuche zur Besiedelung mit Fibroblasten und Charakterisierung des Vliesstoffs wurde wie in D. Grafahrend, J. Lleixa Calvet, J. Salber, P. D. Dalton, M. Möller, D. Klee, Biofunctionalized nanofibers based on resorbable poly(ethylene glycol)-b-polyesters for tissue engineering, J. Mater. Sci. Mater. Med., 2008, 19 (4), 1479-1484 beschrieben durchgeführt. Die Fig. 3 zeigt eine Mikroskopaufnahme des hier beschriebenen Vliesstoffs nach 24 Stunden in Zellkultur und Besiedelung mit humanen Fibroblasten.

Hinsichtlich weiterer vorteilhafter Ausgestaltungen und Weiterbildungen der erfindungsgemäßen Lehre wird einerseits auf den allgemeinen Teil der Beschreibung und andererseits auf die beigefügten Patentansprüche verwiesen.

## Patentansprüche

1. Vliesstoff, umfassend Fasern aus einem Faserrohmaterial, wobei die Fasern zumindest eine biologisch aktive Substanz aufweisen, die zumindest teilweise in und/ oder auf den Fasern angeordnet ist, **dadurch gekennzeichnet, dass** das Faserrohmaterial als polymeren Bestandteil zumindest ein synthetisches Polymer in unmodifizierter und/ oder modifizierter Form aufweist, wobei das synthetische Polymer biologisch abbaubar und/ oder biokompatibel ist.

2. Vliesstoff nach Anspruch 1, **dadurch gekennzeichnet, dass** zumindest ein synthetisches Polymer aus folgender Gruppe ausgewählt ist:
Polylactid (PLA), Polyglycolid (PGA), Polycaprolacton (PCL),
Polyhydroxybuttersäure (PHB), Polydioxanonsäure (PDS),
Polyvinylpyrrolidon (PVP), Polyesteramid (PEA), Polyanhydrid,
Polyorthoester, Polyphosphoester, Polyvinylalkohol (PVA),
Polyethylenoxyd (PEO), Polyethylenglycol (PEG), Polyethylen (PE) oder
Polypropylen (PP), Siliziumverbindungen (z.B. SiO₂) bzw. Copolymere dieser Stoffe, wobei zumindest ein synthetisches Polymer in unmodifizierter und/ oder modifizierter Form vorliegt.

3. Vliesstoff nach einem der voranstehenden Ansprüche, **dadurch gekennzeichnet, dass** das synthetische Polymer aufgrund von funktionellen Gruppen selbst eine biologisch aktive Substanz darstellt.

4. Vliesstoff nach einem der voranstehenden Ansprüche, **dadurch gekennzeichnet, dass** die Fasern miteinander verdrillt sind und/oder eine verdrillte Struktur aufweisen.

5. Vliesstoff nach einem der voranstehenden Ansprüche, **dadurch gekennzeichnet, dass** zumindest ein Teil der Fasern als Nanofasem ausgebildet ist.

6. Vliesstoff nach einem der voranstehenden Ansprüche, **dadurch gekennzeichnet, dass** die biologisch aktive Substanz zumindest teilweise als Beschichtung, Imprägnierung oder Verkapselung vorliegt.

7. Vliesstoff nach einem der voranstehenden Ansprüche, **dadurch gekennzeichnet, dass** zumindest eine biologisch aktive Substanz nanoskalig vorliegt.

8. Vliesstoff nach einem der voranstehenden Ansprüche, **dadurch gekennzeichnet, dass** zumindest eine biologisch aktive Substanz aus folgender Gruppe ausgewählt ist: ein Antiseptikum, ein antimikrobiell wirksamer Stoff, ein Antibiotikum, ein Medikament, ein Wachstumsfaktor, ein Enzym, eine Zelle, ein biologisch aktives Metall oder eine biologisch aktive Metallegierung.

9. Vliesstoff nach einem der voranstehenden Ansprüche, **dadurch gekennzeichnet, dass** die Fasern und/ oder zumindest eine biologisch aktive Substanz oberhalb von 200°C instabil ist.

10. Vliesstoff nach einem der voranstehenden Ansprüche, **dadurch gekennzeichnet, dass** der Vliesstoff eine Reißfestigkeit bei einem spezifischen Flächengewicht von 140 bis 180 g/m² im trockenen Zustand von mindestens 0,10 N/mm² und/ oder eine Reißdehnung in hydratisiertem Zustand von mindestens 100% aufweist.

11. Vliesstoff nach einem der voranstehenden Ansprüche,
**dadurch gekennzeichnet, dass** der Vliesstoff eine offene Porenstruktur mit einer Luftdurchlässigkeit von mindestens 0,5 I/min^{±}cm² aufweist.

12. Verwendung eines Vliesstoffs nach einem der voranstehenden Ansprüche zur Herstellung eines Arzneimittels für die Wundheilung.

13. Medizinprodukt, Arzneimittel, Wundverband, Wattetupfer oder Implantat, enthaltend einen Vliesstoff nach einem der Ansprüche 1 bis 12.

14. Rotationsspinnverfahren zur Herstellung eines Vliesstoffs nach einem der Ansprüche 1 bis 12, wobei das Faserrohmaterial in ein Behältnis gegeben wird, wobei das Behältnis in Rotation versetzt wird und wobei das fluidisierte Faserrohmaterial durch Zentripetalkräfte aus dem Behältnis in Form von Fasern ausgetragen wird.
